# EUROPEAN PATENT APPLICATION

(11) **EP 4 215 516 A1**
(43) Date of publication of application: **26.07.2023**
(21) Application number: 21868988.3
(22) Date of filing: 06.07.2021
(51) Int. Cl.: C07C 23/06, C07C 17/395

(54) **OCTAFLUOROCYCLOBUTANE PURIFICATION METHOD**

(30) Priority: 17.09.2020 JP 2020156607
(71) Applicant: Resonac Corporation, Tokyo 105-8518 (JP)
(72) Inventor: DOU Jun, Tokyo 105-8518 (JP); KURIHARA Hideyuki, Tokyo 105-8518 (JP)
(74) Representative: Strehl Schübel-Hopf & Partner
(86) International application number: PCT/JP2021/025486
(87) International publication number: WO 2022/059301

(57) **Abstract**

Provided is an octafluorocyclobutane purification method capable of removing contained fluorocarbons to yield a highly pure octafluorocyclobutane. The octafluorocyclobutane purification method includes a decomposition step of mixing a crude octafluorocyclobutane containing octafluorocyclobutane and fluorocarbons as impurities with oxygen gas or air to give a mixed gas containing the oxygen gas or air at a concentration of 1% by volume or more and 90% by volume or less and bringing the mixed gas into contact with an impurity decomposing agent containing alumina and an alkaline earth metal compound and to decompose fluorocarbons, at a temperature of 100°C or more and 500°C or less to decompose the fluorocarbons and a concentration step of removing, from the mixed gas in which the fluorocarbons have been decomposed in the decomposition step, the gas containing oxygen gas or air (except octafluorocyclobutane) to increase the concentration of the octafluorocyclobutane.

## Description

### Technical Field

The present invention relates to an octafluorocyclobutane purification method.

### Background Art

Octafluorocyclobutane (FC-C318) is a colorless gas having a boiling point of -5.8°C and is suitably usable as the semiconductor material gas such as an etching gas and a cleaning gas. In recent years, to more accurately form circuit patterns by etching or the like to produce more sophisticated electronic devices, there is a demand for a highly pure etching gas from which impurities are removed as much as possible.

An example method for producing octafluorocyclobutane is a method of purifying an octafluorocyclobutane that is formed as a by-product when tetrafluoroethylene or hexafluoropropene is produced. An octafluorocyclobutane before purification typically contains impurities such as oxygen gas, nitrogen gas, carbon dioxide, and water vapor, and many of these impurities can be removed by distillation.

### Citation List

### Patent Literature

PTL 1: JP 2001-190959 A
PTL 2: JP 2002-212118 A

### Summary of Invention

### Technical Problem

An octafluorocyclobutane before purification further contains, as impurities, fluorocarbons such as perfluorobutane (FC-3110, a boiling point of -2°C), decafluoroisobutane (FC-i3110, a boiling point of 0°C), perfluorocyclobutene (FC-C316, a boiling point of 5°C), and hexafluorobutadiene (FC-316, a boiling point of 6°C) . These fluorocarbons have boiling points close to that of octafluorocyclobutane, and this makes it extremely difficult to produce a highly pure octafluorocyclobutane containing these fluorocarbons at a concentration of less than 1 ppm by mass by distillation. In particular, perfluorobutane and decafluoroisobutane have similar physical properties and molecule sizes to those of octafluorocyclobutane and thus are difficult to separate even with molecular sieves or an adsorbent. In the present invention, fluorocarbons are organic compounds having carbon-fluorine bonds, except octafluorocyclobutane.

PTL 1 discloses a process of decomposing fluorocarbons by using a decomposition reagent. However, if the process disclosed in PTL 1 is applied to purification of the above octafluorocyclobutane, octafluorocyclobutane may be decomposed together with fluorocarbons.

PTL 2 discloses a process in which a crude octafluorocyclobutane containing fluorocarbons as impurities is brought into contact with an impurity decomposing agent containing iron oxide and an alkaline earth metal compound under heating to decompose the fluorocarbons, and the fluorocarbons are removed from the crude octafluorocyclobutane. However, the process disclosed in PTL 2 may insufficiently remove perfluorobutane or decafluoroisobutane.

The present invention is intended to provide an octafluorocyclobutane purification method by which contained fluorocarbons are removed to give a highly pure octafluorocyclobutane.

### Solution to Problem

To solve the problems, aspects of the present invention are the following [1] to [7].
[1] An octafluorocyclobutane purification method of removing a fluorocarbon from a crude octafluorocyclobutane containing octafluorocyclobutane and the fluorocarbon as an impurity, the method including
   a decomposition step of mixing the crude octafluorocyclobutane with oxygen gas or air to give a mixed gas containing the oxygen gas or air at a concentration of 1% by volume or more and 90% by volume or less and bringing the mixed gas into contact with an impurity decomposing agent containing alumina and an alkaline earth metal compound and to decompose the fluorocarbon, at a temperature of 100°C or more and 500°C or less to decompose the fluorocarbon, and
   a concentration step of removing a gas containing the oxygen gas or air (except octafluorocyclobutane) from the mixed gas in which the fluorocarbon has been decomposed in the decomposition step to increase the concentration of the octafluorocyclobutane.
[2] The octafluorocyclobutane purification method according to the aspect [1], in which the alkaline earth metal compound is a carbonate of at least one of magnesium, calcium, strontium, or barium, and the mass ratio of the alumina to the alkaline earth metal compound is 1:9 to 1:1.
[3] The octafluorocyclobutane purification method according to the aspect [1] or [2], in which the impurity decomposing agent further contains an oxide of at least one of copper, tin, nickel, cobalt, chromium, molybdenum, tungsten, or vanadium, and the mass ratio of the total amount of the oxide to the total amount of the alumina and the alkaline earth metal compound is 1:99 to 5:95.
[4] The octafluorocyclobutane purification method according to any one of the aspects [1] to [3], in which the crude octafluorocyclobutane contains the fluorocarbon at a concentration of 1 ppm by mass or more and 10,000 ppm by mass or less.
[5] The octafluorocyclobutane purification method according to any one of the aspects [1] to [4], in which the fluorocarbon is at least one of perfluorobutane, decafluoroisobutane, perfluorocyclobutene, hexafluorobutadiene, tetrafluoromethane, hexafluoroethane, hexafluoropropene, octafluoropropane, octafluorocyclopentene, or fluoroform.
[6] The octafluorocyclobutane purification method according to any one of the aspects [1] to [5], in which the crude octafluorocyclobutane contains the octafluorocyclobutane at a concentration of 10% by volume or more.
[7] The octafluorocyclobutane purification method according to any one of the aspects [1] to [6], in which in the concentration step, the gas containing the oxygen gas or air (except octafluorocyclobutane) is removed by at least one of distillation or membrane separation.

### Advantageous Effects of Invention

According to the present invention, fluorocarbons in a crude octafluorocyclobutane can be removed to give a highly pure octafluorocyclobutane.

### Brief Description of Drawings

FIG. 1 is a schematic view of a purification treatment apparatus used in Example 1 and the like; and
FIG. 2 is a schematic view of a purification treatment apparatus used in Example 20.

### Description of Embodiments

Embodiments of the present invention will now be described. The embodiments are merely examples of the present invention, and the present invention is not limited to the embodiments. Various modifications or improvements can be made in the embodiments, and such modifications and improvements can be encompassed by the present invention.

Octafluorocyclobutane (C₄F₈) having a cyclic structure has relatively high chemical stability. The inventors of the present invention have found that a highly pure octafluorocyclobutane can be produced by removing contained fluorocarbons thanks to the chemical stability of octafluorocyclobutane and have completed the invention.

In other words, an octafluorocyclobutane purification method pertaining to an embodiment of the present invention removes, from a crude octafluorocyclobutane containing octafluorocyclobutane and fluorocarbons as impurities, the fluorocarbons, and the octafluorocyclobutane purification method includes a decomposition step of decomposing the fluorocarbons and a concentration step of increasing the concentration of the octafluorocyclobutane.

The decomposition step is a step in which a crude octafluorocyclobutane is mixed with oxygen gas (O₂) or air to give a mixed gas containing the oxygen gas or air at a concentration of 1% by volume or more and 90% by volume or less, and then the mixed gas is brought into contact with an impurity decomposing agent that decomposes fluorocarbons, at a temperature of 100°C or more and 500°C or less to decompose the fluorocarbons. The impurity decomposing agent contains alumina and an alkaline earth metal compound.

The concentration step is a step in which, from the mixed gas in which the fluorocarbons have been decomposed in the decomposition step, a gas containing the oxygen gas or air (except octafluorocyclobutane) is removed to increase the concentration of octafluorocyclobutane.

An octafluorocyclobutane may contain fluorocarbons as impurities. When a crude octafluorocyclobutane containing octafluorocyclobutane and fluorocarbons is purified by the octafluorocyclobutane purification method pertaining to the present embodiment, the impurity decomposing agent can selectively decompose the fluorocarbons while octafluorocyclobutane is prevented from decomposing, and a highly pure octafluorocyclobutane (hereinafter also called a "purified octafluorocyclobutane") can be produced.

For example, while the decomposition rate of octafluorocyclobutane is suppressed to 1% by mass or less, the concentration of fluorocarbons can be reduced to less than 1 ppm by mass in a purified octafluorocyclobutane. In other words, a purified octafluorocyclobutane can have a purity of 99.999% by mass or more. In particular, perfluorobutane (C₄F₁₀), decafluoroisobutane (C₄F₁₀), and the like, which are insufficiently removed by conventional purification methods such as distillation and adsorption, can be effectively removed. The purity of octafluorocyclobutane can be analyzed by gas chromatography using a thermal conductivity detector (TCD) or a hydrogen flame ionization detector (FID) as the detector.

The highly pure octafluorocyclobutane produced by the octafluorocyclobutane purification method pertaining to the present embodiment is useful, for example, as an etching gas, a cleaning gas, and the like used in a semiconductor production process.

The octafluorocyclobutane purification method pertaining to the present embodiment will now be described in further detail.

### [Crude octafluorocyclobutane]

The crude octafluorocyclobutane to which the octafluorocyclobutane purification method pertaining to the present embodiment is applicable may be any crude octafluorocyclobutane containing fluorocarbons as impurities, and the octafluorocyclobutane purification method pertaining to the present embodiment can be applied to an octafluorocyclobutane produced by a known method or to a commercially available octafluorocyclobutane.

In the crude octafluorocyclobutane, the concentration of fluorocarbons is preferably 1 ppm by mass or more and 10,000 ppm by mass or less, more preferably 1 ppm by mass or more and 1,000 ppm by mass or less, and even more preferably 1 ppm by mass or more and 100 ppm by mass or less. When the crude octafluorocyclobutane contains fluorocarbons at a concentration within the above range, the fluorocarbons can be decomposed at a temperature at which octafluorocyclobutane is more unlikely to decompose.

In the crude octafluorocyclobutane, the concentration of octafluorocyclobutane is preferably 10% by volume or more, more preferably 20% by volume or more, and even more preferably 50% by volume or more. When the crude octafluorocyclobutane contains octafluorocyclobutane at a concentration within the above range, reaction heat is suppressed advantageously. Examples of the components contained in the crude octafluorocyclobutane include, in addition to octafluorocyclobutane and fluorocarbons, oxygen gas and nitrogen gas (N₂).

### [Fluorocarbons]

The fluorocarbons contained as impurities in the crude octafluorocyclobutane may be any organic compounds having carbon-fluorine bonds and excluding octafluorocyclobutane, but the octafluorocyclobutane purification method pertaining to the present embodiment is particularly preferably used to remove the following fluorocarbons.

In other words, examples of the fluorocarbons include perfluorobutane, decafluoroisobutane, perfluorocyclobutene (C₄F₆), hexafluorobutadiene (C₄F₆), tetrafluoromethane (FC-14, CF₄), hexafluoroethane (FC-116, C₂F₆), hexafluoropropene (FC-216, C₃F₆), octafluoropropane (FC-218, C₃F₈), octafluorocyclopentene (FC-418, C₅F₈), and fluoroform (HFC-23, CHF₃).

The crude octafluorocyclobutane may contain a single fluorocarbon or two or more fluorocarbons.

### [Impurity decomposing agent]

The impurity decomposing agent used in the octafluorocyclobutane purification method pertaining to the present embodiment contains alumina and an alkaline earth metal compound. When the impurity decomposing agent contains alumina and an alkaline earth metal compound, fluorocarbons are selectively and efficiently decomposed while octafluorocyclobutane is prevented from decomposing.

The alumina may be any type, but to adsorb a larger amount of gas, an alumina having a specific surface area of 50 m²/g or more is preferred, and an alumina having a specific surface area of 100 m²/g or more and 300 m²/g or less is more preferred.

The alumina is preferably in a powder form. To be uniformly mixed with other substances, alumina particles preferably have an average particle size of more than 0.1 µm and not more than 100 µm or less, and the upper limit is more preferably 30 µm or less and even more preferably 5 µm or less. In the present invention, the average particle size is a d50 value in volume distribution, corresponds to a median value that bisects the area under a frequency curve, and can be determined by particle size measurement by laser diffraction.

The content of alkali metals (such as sodium and potassium) contained as impurities in the alumina is preferably 0.1% by mass or less, more preferably 0.01% by mass or less, and even more preferably 0.001% by mass or less to achieve the reactivity with fluorocarbons.

The alkaline earth metal compound may be any type but is preferably a carbonate of at least one of magnesium, calcium, strontium, and barium and is more preferably a carbonate of calcium.

When the alkaline earth metal compound is calcium carbonate (CaCO₃), fluorine gas formed by decomposition of fluorocarbons is reacted with calcium carbonate and is fixed as calcium fluoride (CaF₂), and thus fluorination of alumina is prevented. As a result, alumina is likely to maintain the performance (activity) of decomposing fluorocarbons.

The alkaline earth metal compound is preferably in a powder form. To be uniformly mixed with other substances, alkaline earth metal compound particles preferably have an average particle size of 1 µm or more and 100 µm or less, and the upper limit is more preferably 30 µm or less and even more preferably 5 µm or less.

The content of alkali metals (such as sodium and potassium) contained as impurities in the alkaline earth metal compound is preferably 0.1% by mass or less, more preferably 0.01% by mass or less, and even more preferably 0.001% by mass or less to achieve the reactivity with fluorocarbons.

The impurity decomposing agent contains the alkaline earth metal compound in an amount equal to the amount of alumina or more than the amount of alumina, and the mass ratio of the alumina to the alkaline earth metal compound is preferably 1:9 to 1:1 and more preferably 1:4 to 2:3. When the proportion of the amount of the alumina to the total amount of the alumina and the alkaline earth metal compound is 10% by mass or more, the catalyst has a larger number of active sites to improve the performance of decomposing fluorocarbons. When the proportion of the amount of the alumina to the total amount of the alumina and the alkaline earth metal compound is 50% by mass or less, the content of the alkaline earth metal compound is 50% by mass or more to improve the effective utilization factor of the impurity decomposing agent.

The impurity decomposing agent used in the octafluorocyclobutane purification method pertaining to the present embodiment may further contain an oxide of at least one of copper, tin, nickel, cobalt, chromium, molybdenum, tungsten, and vanadium as needed. Of these metal oxides, copper oxides (Cu₂O, CuO), tin oxides (SnO, SnO₂), and vanadium oxide (V₂O₅) are more preferred, and copper oxides and tin oxides are even more preferred.

When the impurity decomposing agent contains the above oxide, the oxide functions as a catalytic promoter to improve the fluorocarbon decomposing performance of the impurity decomposing agent. In addition, carbon monoxide generated by decomposition of fluorocarbons is oxidized into carbon dioxide, and thus the generation amount of carbon monoxide can be suppressed.

The oxide is preferably in a powder form. To be uniformly mixed with other substances, the oxide particles preferably have an average particle size of 1 µm or more and 100 µm or less, and the upper limit is more preferably 30 µm or less and even more preferably 5 µm or less.

The content of alkali metals (such as sodium and potassium) contained as impurities in the oxide is preferably 0.1% by mass or less, more preferably 0.01% by mass or less, and even more preferably 0.001% by mass or less to achieve the reactivity with fluorocarbons.

When the impurity decomposing agent contains the oxide, the total amount of the oxide may be less than the total amount of the alumina and the alkaline earth metal compound, and the mass ratio of the total amount of the oxide contained in the impurity decomposing agent to the total amount of the alumina and the alkaline earth metal compound is preferably 1:99 to 5:95.

When the proportion of the amount of the oxide to the total amount of the oxide, the alumina, and the alkaline earth metal compound is 1% by mass or more, the effect of the oxide is sufficiently exerted. If the proportion of the amount of the oxide to the total amount of the oxide, the alumina, and the alkaline earth metal compound is more than 5% by mass, the effect of the oxide is saturated. When the proportion is 5% by mass or less, the total amount of the alumina and the alkaline earth metal compound is 95% by mass or more to improve the effective utilization factor of the impurity decomposing agent.

The impurity decomposing agent may be in any form but is preferably in a powder form, a particulate form, a pellet form, a spherical form, or the like. The impurity decomposing agent in such a form preferably has an average particle size of 0.5 mm or more and 10 mm or less and more preferably 1 mm or more and 5 mm or less. When having an average particle size within the above range, the impurity decomposing agent has an appropriate surface area, which contributes to the adsorption-diffusion of a mixed gas, and thus a mixed gas to be purified is likely to have a preferred diffusion speed.

The method of producing the impurity decomposing agent will next be described with reference to examples . The method of producing the impurity decomposing agent is not limited to particular methods, but the impurity decomposing agent can be produced by mixing an alumina powder with an alkaline earth metal compound powder. When the powders are mixed, adding a binder facilitates granulation even when the powders have different average particle sizes . The binder may be any type, but, for example, alumina fine powder or alum is preferred.

Adding alumina fine powder as the binder improves the uniform electrodeposition between an alumina powder and an alkaline earth metal compound powder and facilitates granulation of the alkaline earth metal compound powder. The alumina fine powder as the binder preferably has an average particle size of 0.1 µm or less and more preferably 0.05 µm or less.

The alum is preferably in a powder form, and the alum powder preferably has an average particle size of 0.1 µm or less. The content of alkali metals (such as sodium and potassium) contained as impurities in the alum is preferably 0.1% by mass or less and more preferably 0.01% by mass or less to suppress a reduction in the number of reaction sites on the alumina surface and to suppress a reduction in the fluorocarbon decomposing performance of the impurity decomposing agent.

To produce the impurity decomposing agent by mixing an alumina powder and an alkaline earth metal compound powder, first, water is added to the powders, and the powders are kneaded and pulverized into granules, giving a particulate matter. Next, the particulate matter is dried, for example in an inert gas such as nitrogen or in air at a temperature of 100°C or more and 200°C or less, and water is removed from the particulate matter. The dried particulate matter is then burned to give a particulate impurity decomposing agent.

Burning improves the hardness, and thus the particulate impurity decomposing agent is prevented from breaking or pulverizing at the time of operation such as packing the particulate impurity decomposing agent in a reactor.

The burning temperature is preferably 400°C or more and 1,000°C or less, more preferably 400°C or more and 700°C or less, and even more preferably 500°C or more and 700°C or less. Burning at such a temperature makes alumina into pseudoboehmite alumina. At a temperature within the above range, the alkaline earth metal compound is unlikely to decompose, or the activity of the impurity decomposing agent is unlikely to deteriorate.

### [Decomposition step]

In the decomposition step, first, a crude octafluorocyclobutane is mixed with oxygen gas or air to give a mixed gas. In the step, the concentration of oxygen gas or air in the mixed gas is 1% by volume or more and 90% by volume or less and preferably 10% by volume or more and 90% by volume or less.

When the fluorocarbon contained as the impurity in the crude octafluorocyclobutane is perfluorobutane, and the alkaline earth metal compound in the impurity decomposing agent is calcium carbonate, the decomposition reaction formula is as below. In the reaction, carbon monoxide may be generated in addition to carbon dioxide, but as mentioned above, carbon monoxide is converted in the presence of the above oxide into carbon dioxide. The carbon dioxide gas generated by the reaction is removed in the subsequent concentration step, and calcium fluoride is removed as a solid precipitate.

5CaCO₃ + C₄F₁₀ + (3/2)O₂ → 5CaF₂ + 9CO₂

When a carbonate of magnesium, strontium, or barium, other than calcium carbonate, is used as the alkaline earth metal compound, or when fluorocarbons other than perfluorobutane are decomposed, a similar reaction proceeds to generate a metal fluoride, carbon dioxide, and the like. When a fluorocarbon has hydrogen atoms, water vapor (H₂O) is generated, and when a fluorocarbon has chlorine atoms, a metal chloride is generated as a solid precipitate. Oxygen gas in an amount equivalent to the reaction is needed, but when the content of fluorocarbons as impurities is about 1,000 ppm by mass or less, the mixed gas can contain oxygen gas or air at a concentration of 1% by volume or more.

When the mixed gas contains oxygen gas or air at a concentration of 1% by volume or more, decomposition of fluorocarbons does not generate excess reaction heat, and the impurity decomposing agent achieves enough activity. Hence, fluorocarbons can be sufficiently decomposed. When containing oxygen gas or air at a concentration of 90% by volume or less, the mixed gas contains a crude octafluorocyclobutane at a high concentration, and thus the crude octafluorocyclobutane can be efficiently purified. When the mixed gas contains oxygen gas or air at a concentration of more than 90% by volume, octafluorocyclobutane is likely to decompose, and thus such a condition is unfavorable.

Next, the mixed gas is brought into contact with an impurity decomposing agent at a temperature of 100°C or more and 500°C or less to decompose fluorocarbons. When the mixed gas is brought into contact with the impurity decomposing agent at a temperature of 100°C or more, the impurity decomposing agent achieves enough activity, and thus fluorocarbons can be sufficiently decomposed. At a temperature of 500°C or less, decomposition of octafluorocyclobutane by the impurity decomposing agent is suppressed. The temperature when the mixed gas is brought into contact with the impurity decomposing agent is required to be 100°C or more and 500°C or less and is preferably 250°C or more and 500°C or less.

The pressure condition when the mixed gas is brought into contact with the impurity decomposing agent is not limited to particular conditions and may be any of an atmospheric pressure condition, a pressurized condition, and a vacuum condition, but is preferably -0.1 MPaG or more and 0.3 MPaG or less and more preferably 0 MPaG or more and 0.2 MPaG or less. At 0.3 MPaG or less, octafluorocyclobutane is unlikely to liquefy.

The purification treatment speed of a crude octafluorocyclobutane can be expressed by the space velocity of a mixed gas coming into contact with an impurity decomposing agent. The space velocity of the mixed gas coming into contact with the impurity decomposing agent is preferably 1 h⁻¹ or more and 300 h⁻¹ or less and more preferably 10 h⁻¹ or more and 150 h⁻¹ or less. At a velocity within the above range, fluorocarbons can be efficiently decomposed, and a purified octafluorocyclobutane containing fluorocarbons at a concentration of less than 1 ppm by mass is more easily produced.

The mixed gas is brought into contact with the impurity decomposing agent by any method, and an example is a method of supplying a crude octafluorocyclobutane to a reactor filled with the impurity decomposing agent. For example, a method of continuously allowing the mixed gas to pass through a fixed bed with the impurity decomposing agent can be adopted.

### [Concentration step]

The concentration step is a step of removing, from the mixed gas in which fluorocarbons have been decomposed in the decomposition step, the gas containing oxygen gas or air (except octafluorocyclobutane) to increase the concentration of octafluorocyclobutane.

The mixed gas in which fluorocarbons have been decomposed in the decomposition step contains nitrogen gas, oxygen gas, and carbon dioxide and may contain water vapor and the like in some cases, and thus by removing these gases to increase the concentration of octafluorocyclobutane in the concentration step, a purified octafluorocyclobutane, for example, having a purity of 99.999% by mass or more is produced.

The method of removing nitrogen gas, oxygen gas, carbon dioxide, and the like may be any method, and distillation or membrane separation may be adopted.

As example distillation conditions in the distillation, the temperature of a low-boiling component cutting distillation column is -80°C or more and 70°C or less, and the operating pressure is -0.1 MPaG or more and 1.0 MPaG or less.

The type of the separation membrane used in the membrane separation may be any membrane through which nitrogen gas, oxygen gas, carbon dioxide, and the like easily pass, but octafluorocyclobutane is difficult to pass (more preferably, almost no octafluorocyclobutane passes), and, for example, a membrane formed from an aromatic polyimide, silicone hollow fibers, or porous carbon fibers is preferred.

The environmental temperature during separation by the membrane separation is preferably set at 0°C or more and 100°C or less and more preferably 20°C or more and 80°C or less. At an environmental temperature of 0°C or more, octafluorocyclobutane, which has a high vapor pressure, is concentrated at a high speed. At an environmental temperature of 100°C or less, a separation membrane is unlikely to deteriorate.

The pressure when the mixed gas in which fluorocarbons have been decomposed in the decomposition step is supplied to a separation membrane is preferably set at 0 MPaG or more and 1 MPaG or less and more preferably 0 MPaG or more and 0.5 MPaG or less. At 1 MPaG or less, a high temperature to prevent octafluorocyclobutane from liquifying is not needed, and thus a separation membrane is unlikely to deteriorate.

### Examples

The present invention will next be described more specifically with reference to examples and comparative examples.

### [Example 1]

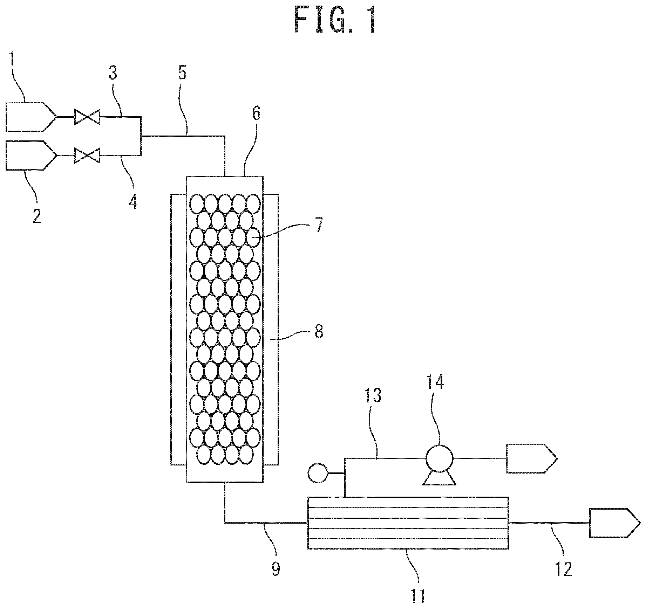

By using a purification treatment apparatus in FIG. 1 in which the concentration step was performed by membrane separation, a crude octafluorocyclobutane containing octafluorocyclobutane and perfluorobutane as the impurity was purified. The crude octafluorocyclobutane contained perfluorobutane at a concentration of 100 ppm by mass.

An impurity decomposing agent used in Example 1 will be described. First, 30 parts by mass of alumina (an average particle size of 5 µm), 70 parts by mass of calcium carbonate, 5 parts by mass of copper oxide, and 0.1 part by mass of alumina fine powder (an average particle size of 0.1 µm) were mixed in a total amount of 30 g, and the whole was mixed in a Henschel Mixer. To the mixture, 10 mL of water was added, and the mixture was granulated. The granules were dried at 110°C for 3 hours and sieved to give a particulate impurity decomposing agent 7 having a particle size of 0.85 to 2.8 mm.

Next, the structure of the purification treatment apparatus in FIG. 1 and a crude octafluorocyclobutane purification method using the purification treatment apparatus in FIG. 1 will be described.

The purification treatment apparatus in FIG. 1 includes a crude octafluorocyclobutane container 1 filled with a crude octafluorocyclobutane, an air container 2 filled with air, a tubular reactor 6 containing the impurity decomposing agent 7, a heater 8 to heat the reactor 6, and a membrane separation unit 11 including separation membranes (not illustrated).

The reactor 6 is a quartz tube having an inner diameter of 38 mm and a length of 1,000 mm and contains 350 g of the impurity decomposing agent 7. The impurity decomposing agent 7 is burned in the reactor 6 before use for purification of a crude octafluorocyclobutane. In other words, while allowing nitrogen gas to pass through the reactor 6, the impurity decomposing agent 7 was heated at 350°C for 12 hours and at 600°C for 3 hours to be burned. The nitrogen gas was introduced from the upper side of the reactor 6 and discharged from the lower side (not illustrated).

The crude octafluorocyclobutane in the crude octafluorocyclobutane container 1 was supplied through a crude octafluorocyclobutane pipe 3 to a mixed gas supply pipe 5 while the flow rate was controlled by using a mass flow controller or the like. The air in the air container 2 was supplied through an air pipe 4 to the mixed gas supply pipe 5 while the flow rate was controlled by using a mass flow controller or the like. Accordingly, the crude octafluorocyclobutane and the air were mixed in the mixed gas supply pipe 5 to give a mixed gas having a volume ratio of crude octafluorocyclobutane to air of 50/50 (the concentration of air in the mixed gas was 50% by volume, and both the volume ratio and the concentration are illustrated in Table 1).

The mixed gas was supplied through the mixed gas supply pipe 5 to the reactor 6 and was brought into contact with the impurity decomposing agent 7 at 400°C controlled by the heater 8. The pressure in the reactor 6 was normal pressure (0 MPaG) . The space velocity of the mixed gas was 15 Hh⁻¹, the linear velocity was 0.074 m/min, and the residence time was 4 minutes . Accordingly, perfluorobutane contained in the crude octafluorocyclobutane was decomposed (decomposition step).

Next, the mixed gas in which perfluorobutane had been decomposed was supplied through a decomposition gas pipe 9 to the membrane separation unit 11 and was allowed to pass through a tubular separation membrane (not illustrated) at room temperature. The separation membrane was made from an aromatic polyimide and had a length of 1 m. By using a vacuum pump 14 to reduce the pressure on the permeation side of the separation membrane to -0.1 MPaG, components other than octafluorocyclobutane (nitrogen gas, oxygen gas, and the like) passed through the separation membrane and flowed into a permeation side pipe 13.

The octafluorocyclobutane did not pass through the separation membrane and flowed into a non-permeation side pipe 12. The concentration of perfluorobutane in the octafluorocyclobutane from the non-permeation side pipe 12 was determined by gas chromatography and was less than 0.1 ppm by mass. The rate of octafluorocyclobutane decomposed during the purification(decomposition rate) was determined by gas chromatography and was less than 1% by mass. The decomposition rate of octafluorocyclobutane decomposed during purification was calculated in accordance with the following equation.

Decomposition rate (%) = 100 × {(molar amount of octafluorocyclobutane before purification step) - (molar amount of octafluorocyclobutane after purification step)}/(molar amount of octafluorocyclobutane before purification step)

**[Table 1]**

| | Alumina/calcium carbonate mass ratio in impurity decomposing agent | Volume ratio of crude octafluorocyclobutane to air*) | Concentration of perfluorobutane in crude octafluorocyclobutane (ppm by mass) | Decomposition temperature (°C) | Concentration of perfluorobutane in purified octafluorocyclobutane (ppm by mass) | Octafluorocyclobutane decomposition rate (%) |
|---|---|---|---|---|---|---|
| Ex. 1 | 30/70 | 50/50 (50% by volume) | 100 | 400 | less than 0.1 | less than 1 |
| Ex. 2 | 30/70 | 50/50 (50% by volume) | 100 | 300 | 0.3 | less than 1 |
| Ex. 3 | 30/70 | 50/50 (50% by volume) | 100 | 200 | 0.9 | less than 1 |
| Ex. 4 | 30/70 | 50/50 (50% by volume) | 1 | 100 | less than 0.1 | less than 1 |
| Ex. 5 | 30/70 | 10/90 (90% by volume) | 10000 | 500 | less than 0.1 | less than 1 |
| Ex. 6 | 30/70 | 99/1 (1% by volume) | 1 | 500 | less than 0.1 | less than 1 |
| Ex. 7 | 10/90 | 50/50 (50% by volume) | 100 | 400 | less than 0.1 | less than 1 |
| Ex. 8 | 50/50 | 50/50 (50% by volume) | 100 | 400 | less than 0.1 | less than 1 |
| Comp. Ex. 1 | 30/70 | 50/50 (50% by volume) | 100 | 600 | less than 0.1 | 33 |
| Comp. Ex. 2 | 30/70 | 5/95 (95% by volume) | 100 | 400 | less than 0.1 | 8 |

| | | | | | | |
|---|---|---|---|---|---|---|
| *) In parentheses, the concentration of air in a mixed gas is illustrated. | | | | | | |

### [Examples 2 to 8 and Comparative Examples 1 and 2]

The same procedure as in Example 1 was performed except that the alumina/calcium carbonate mass ratio in an impurity decomposing agent, the volume ratio of crude octafluorocyclobutane to air, the concentration of perfluorobutane in a crude octafluorocyclobutane, and the decomposition temperature were set as illustrated in Table 1, purifying a crude octafluorocyclobutane. The results (the concentration of perfluorobutane in the purified octafluorocyclobutane and the octafluorocyclobutane decomposition rate) are illustrated in Table 1.

### [Example 9]

The same procedure as in Example 1 was performed except that oxygen gas was mixed with the crude octafluorocyclobutane in place of air to give a mixed gas, purifying the crude octafluorocyclobutane. The results (the concentration of perfluorobutane in the purified octafluorocyclobutane and the octafluorocyclobutane decomposition rate) are illustrated in Table 2.

### [Example 10]

The same procedure as in Example 4 was performed except that oxygen gas was mixed with the crude octafluorocyclobutane in place of air to give a mixed gas, purifying the crude octafluorocyclobutane. The results (the concentration of perfluorobutane in the purified octafluorocyclobutane and the octafluorocyclobutane decomposition rate) are illustrated in Table 2.

**[Table 2]**

| | Alumina/calcium carbonate mass ratio in impurity decomposing agent | Volume ratio of crude octafluorocyclobutane to oxygen gas*) | Concentration of perfluorobutane in crude octafluorocyclobutane (ppm by mass) | Decomposition temperature (°C) | Concentration of perfluorobutane in purified octafluorocyclobutane (ppm by mass) | Octafluorocyclobutane decomposition rate (%) |
|---|---|---|---|---|---|---|
| Ex. 9 | 30/70 | 50/50 (50% by volume) | 100 | 400 | less than 0.1 | less than 1 |
| Ex. 10 | 30/70 | 50/50 (50% by volume) | 1 | 100 | less than 0.1 | less than 1 |

| | | | | | | |
|---|---|---|---|---|---|---|
| *) In parentheses, the concentration of oxygen in a mixed gas is illustrated. | | | | | | |

### [Example 11]

The same procedure as in Example 1 was performed except that the fluorocarbon as the impurity in the crude octafluorocyclobutane was decafluoroisobutane, purifying the crude octafluorocyclobutane. As a result, the concentration of decafluoroisobutane in the purified octafluorocyclobutane was less than 0.1 ppm by mass, and the octafluorocyclobutane decomposition rate was less than 1% by mass.

### [Example 12]

The same procedure as in Example 1 was performed except that the fluorocarbon as the impurity in the crude octafluorocyclobutane was perfluorocyclobutene, purifying the crude octafluorocyclobutane. As a result, the concentration of perfluorocyclobutene in the purified octafluorocyclobutane was less than 0.1 ppm by mass, and the octafluorocyclobutane decomposition rate was less than 1% by mass.

### [Example 13]

The same procedure as in Example 1 was performed except that the fluorocarbon as the impurity in the crude octafluorocyclobutane was hexafluorobutadiene, purifying the crude octafluorocyclobutane. As a result, the concentration of hexafluorobutadiene in the purified octafluorocyclobutane was less than 0.1 ppm by mass, and the octafluorocyclobutane decomposition rate was less than 1% by mass.

### [Example 14]

The same procedure as in Example 1 was performed except that the fluorocarbon as the impurity in the crude octafluorocyclobutane was tetrafluoromethane, purifying the crude octafluorocyclobutane. As a result, the concentration of tetrafluoromethane in the purified octafluorocyclobutane was less than 0.1 ppm by mass, and the octafluorocyclobutane decomposition rate was less than 1% by mass.

### [Example 15]

The same procedure as in Example 1 was performed except that the fluorocarbon as the impurity in the crude octafluorocyclobutane was hexafluoroethane, purifying the crude octafluorocyclobutane. As a result, the concentration of hexafluoroethane in the purified octafluorocyclobutane was less than 0.1 ppm by mass, and the octafluorocyclobutane decomposition rate was less than 1% by mass.

### [Example 16]

The same procedure as in Example 1 was performed except that the fluorocarbon as the impurity in the crude octafluorocyclobutane was hexafluoropropene, purifying the crude octafluorocyclobutane. As a result, the concentration of hexafluoropropene in the purified octafluorocyclobutane was less than 0.1 ppm by mass, and the octafluorocyclobutane decomposition rate was less than 1% by mass.

### [Example 17]

The same procedure as in Example 1 was performed except that the fluorocarbon as the impurity in the crude octafluorocyclobutane was octafluoropropane, purifying the crude octafluorocyclobutane. As a result, the concentration of octafluoropropane in the purified octafluorocyclobutane was less than 0.1 ppm by mass, and the octafluorocyclobutane decomposition rate was less than 1% by mass.

### [Example 18]

The same procedure as in Example 1 was performed except that the fluorocarbon as the impurity in the crude octafluorocyclobutane was octafluorocyclopentene, purifying the crude octafluorocyclobutane. As a result, the concentration of octafluorocyclopentene in the purified octafluorocyclobutane was less than 0.1 ppm by mass, and the octafluorocyclobutane decomposition rate was less than 1% by mass.

### [Example 19]

The same procedure as in Example 1 was performed except that the fluorocarbon as the impurity in the crude octafluorocyclobutane was fluoroform, purifying the crude octafluorocyclobutane. As a result, the concentration of fluoroform in the purified octafluorocyclobutane was less than 0.1 ppm by mass, and the octafluorocyclobutane decomposition rate was less than 1% by mass.

### [Example 20]

The same procedure as in Example 1 was performed except that a concentration step by distillation was performed in place of the concentration step by membrane separation, purifying the crude octafluorocyclobutane. The result (the purity of the purified octafluorocyclobutane) is illustrated in Table 3.

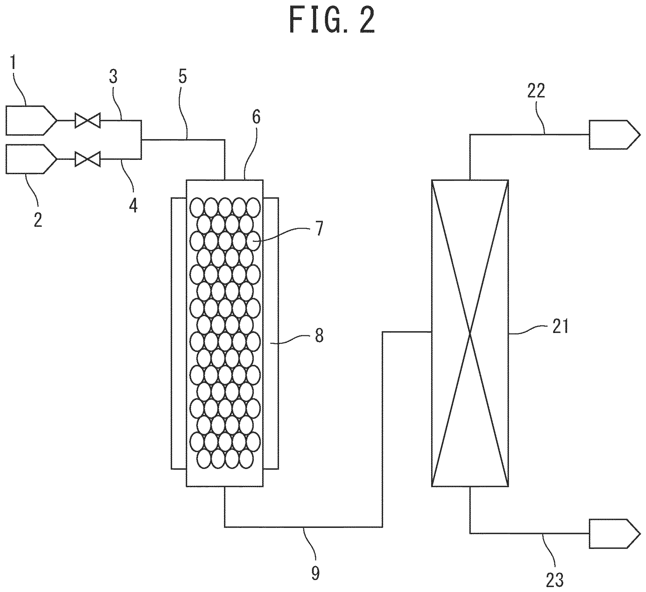

In Example 20, the crude octafluorocyclobutane was purified by using a purification treatment apparatus in FIG. 2 in which the concentration step was performed by distillation. The structure of the purification treatment apparatus in FIG. 2 and the crude octafluorocyclobutane purification method using the purification treatment apparatus in FIG. 2 will be described below. The structure of the purification treatment apparatus in FIG. 2 is substantially the same as the structure of the purification treatment apparatus in FIG. 1 except units for the concentration step, and thus the same units are not described. In FIG. 2, the same units as in the purification treatment apparatus in FIG. 1 are indicated by the same signs as in FIG. 1.

First, the decomposition step was performed in the same manner as in Example 1 to decompose perfluorobutane contained in a crude octafluorocyclobutane. Next, the mixed gas in which perfluorobutane had been decomposed was supplied through a decomposition gas pipe 9 to a distillation column 21 and was distilled. The distillation column 21 had a diameter of 100 mm and a stage number of 10. The filler packed in the distillation column 21 was Cascade Mini-Rings (registered trademark) No. 0P manufactured by Matsui Machine, and the packing height was 2 m. As the distillation conditions, the filler temperature was 1°C, the operating pressure was 0.05 MPaG, and the cutting rate was 5% by mass.

When distillation was performed in the above conditions, low-boiling components flowed into a low-boiling pipe 22 connected to the column top, whereas a high-boiling component flowed into a high-boiling pipe 23 connected the column bottom. The high-boiling component discharged from the high-boiling pipe 23 was a purified octafluorocyclobutane. The concentration of perfluorobutane in the purified octafluorocyclobutane was determined by gas chromatography and was less than 0.1 ppm by mass. In other words, the purified octafluorocyclobutane had a purity of 99.999% by mass.

**[Table 3]**

| | Alumina/calcium carbonate mass ratio in impurity decomposing agent | Volume ratio of crude octafluorocyclobutane to air*) | Concentration of perfluorobutane in crude octafluorocyclobutane (ppm by mass) | Concentration of perfluorobutane in purified octafluorocyclobutane (ppm by mass) | Octafluorocyclobutane decomposition rate (%) | Concentration step | Purity of purified octafluorocyclobutane (% by mass) |
|---|---|---|---|---|---|---|---|
| Ex. 1 | 30/70 | 50/50 (50% by volume) | 100 | less than 0.1 | less than 1 | Membrane separation | 99.999 |
| Ex. 20 | 30/70 | 50/50 (50% by volume) | 100 | less than 0.1 | less than 1 | Distillation | 99.999 |
| Comp. Ex. 3 | 30/70 | 50/50 (50% by volume) | 100 | less than 0.1 | less than 1 | None | 48.937 |

| | | | | | | | |
|---|---|---|---|---|---|---|---|
| *) In parentheses, the concentration of air in a mixed gas is illustrated. | | | | | | | |

### [Comparative Example 3]

The same procedure as in Example 1 was performed except that the concentration step by membrane separation was not performed, purifying the crude octafluorocyclobutane. The result (the purity of the purified octafluorocyclobutane) is illustrated in Table 3.

### Reference Signs List

- 1: crude octafluorocyclobutane container
- 2: air container
- 6: reactor
- 7: impurity decomposing agent
- 11: membrane separation unit
- 21: distillation column

## Claims

1. An octafluorocyclobutane purification method of removing a fluorocarbon from a crude octafluorocyclobutane containing octafluorocyclobutane and the fluorocarbon as an impurity, the method comprising:
a decomposition step of mixing the crude octafluorocyclobutane with oxygen gas or air to give a mixed gas containing the oxygen gas or air at a concentration of 1% by volume or more and 90% by volume or less and bringing the mixed gas into contact with an impurity decomposing agent containing alumina and an alkaline earth metal compound and to decompose the fluorocarbon, at a temperature of 100°C or more and 500°C or less to decompose the fluorocarbon; and
a concentration step of removing a gas containing the oxygen gas or air (except octafluorocyclobutane from the mixed gas in which the fluorocarbon has been decomposed in the decomposition step to increase a concentration of the octafluorocyclobutane.

2. The octafluorocyclobutane purification method according to claim 1, wherein the alkaline earth metal compound is a carbonate of at least one of magnesium, calcium, strontium, or barium, and a mass ratio of the alumina to the alkaline earth metal compound is 1:9 to 1:1.

3. The octafluorocyclobutane purification method according to claim 1 or claim 2, wherein the impurity decomposing agent further contains an oxide of at least one of copper, tin, nickel, cobalt, chromium, molybdenum, tungsten, or vanadium, and a mass ratio of a total amount of the oxide to a total amount of the alumina and the alkaline earth metal compound is 1:99 to 5:95.

4. The octafluorocyclobutane purification method according to any one of claims 1 to 3, wherein the crude octafluorocyclobutane contains the fluorocarbon at a concentration of 1 ppm by mass or more and 10,000 ppm by mass or less.

5. The octafluorocyclobutane purification method according to any one of claims 1 to 4, wherein the fluorocarbon is at least one of perfluorobutane, decafluoroisobutane, perfluorocyclobutene, hexafluorobutadiene, tetrafluoromethane, hexafluoroethane, hexafluoropropene, octafluoropropane, octafluorocyclopentene, or fluoroform.

6. The octafluorocyclobutane purification method according to any one of claims 1 to 5, wherein the crude octafluorocyclobutane contains the octafluorocyclobutane at a concentration of 10% by volume or more.

7. The octafluorocyclobutane purification method according to any one of claims 1 to 6, wherein in the concentration step, the gas containing the oxygen gas or air (except octafluorocyclobutane) is removed by at least one of distillation or membrane separation.
